# EUROPEAN PATENT APPLICATION

(11) **EP 4 613 304 A1**
(43) Date of publication of application: **10.09.2025**
(21) Application number: 23886137.1
(22) Date of filing: 26.10.2023
(51) Int. Cl.: A61M 15/00, A61M 11/00

(54) **INHALER**

(30) Priority: 01.11.2022 KR 20220143733
(71) Applicant: KT&G Corporation, Daedeok-gu Daejeon 34337 (KR)
(72) Inventor: KIM, Moonwon, Daejeon 34128 (KR); JUNG, Yongmi, Daejeon 34128 (KR); SHIN, Jun Won, Daejeon 34128 (KR)
(74) Representative: Ter Meer Steinmeister & Partner
(86) International application number: PCT/KR2023/016769
(87) International publication number: WO 2024/096441

(57) **Abstract**

An inhaler according to an embodiment may comprise: a body which includes a first surface and a second surface opposite to the first surface and provided with a mouthpiece, and in which a first channel and a second channel partitioned from each other in the longitudinal direction from the first surface toward the second surface are formed; a cartridge which is coupled to the first surface of the body to communicate with the first channel and the second channel, and in which a plurality of inhalable compositions are contained; and a first baffle set and a second baffle set which are disposed in the first channel and the second channel respectively, wherein, when a suction force is applied to the mouthpiece, the first baffle set and the second baffle set each control the particle sizes of the plurality of inhalable compositions transferred to the mouthpiece.

## Description

### TECHNICAL FIELD

An inhaler is disclosed.

### BACKGROUND ART

In general, an inhaler is a device used to inhale a composition such as a drug or other substance as a liquid or gas through an oral cavity or nasal cavity during an inhalation process. Such an inhaler is equipped with a container that accommodates an inhalable composition, and the composition may be sprayed from the container through a tube connected to the container and finally into the oral cavity or nasal cavity of a user through an intake.

In addition to a liquid inhaler and a vapor inhaler, such an inhaler includes a powder inhaler that sprays fine powder of the composition as microparticles and an aerosol inhaler that supplies the composition by aerosolizing the composition.

Recently, the application fields of dry powder inhalers have expanded, and the scope has expanded to not only disposable or multi-use powder inhalers, but also powder inhalers that inhale medicinal substances, functional substances, nicotine, and other preference products.

The above description is information the inventor(s) acquired during the course of conceiving the present disclosure or already possessed at the time and is not necessarily art publicly known before the present application was filed.

Prior art document: Korea Patent Publication No. 10-1759972

### DISCLOSURE OF THE INVENTION

### TECHNICAL GOALS

Embodiments are to provide an inhaler that may transfer an inhalable composition by utilizing an inertial force of particles and may prevent inflow of particles of an unspecified size that may cause coughing.

Embodiments are to provide an inhaler that may transfer a plurality of inhalable compositions by arranging, on both sides, baffle structures with different performances of removing particles.

The technical goals obtainable from the embodiments are not limited to the above-mentioned technical goals, and other unmentioned technical goals may be clearly understood from the following description by one of ordinary skill in the art to which the present disclosure pertains.

### TECHNICAL SOLUTIONS

An inhaler according to an embodiment for achieving the goals includes a main body that includes a first surface and a second surface facing the first surface and having a mouthpiece and has a first channel and a second channel formed inside and partitioned in a longitudinal direction from the first surface toward the second surface, a cartridge coupled to the first surface of the main body, communicating with the first channel and the second channel, and containing a plurality of inhalable compositions, and a first baffle set arranged in the first channel and a second baffle set arranged in the second channel, wherein, when a suction force is applied to the mouthpiece, the first baffle set and the second baffle set control a particle size of the plurality of inhalable compositions transferred to the mouthpiece.

The first baffle set or the second baffle set may each include a plurality of baffles, wherein the plurality of baffles of the first baffle set is arranged to be spaced apart alternately on an inner wall of the first channel in the longitudinal direction and the plurality of baffles of the second baffle set is arranged to be spaced apart alternately on an inner wall of the second channel in the longitudinal direction.

According to an aspect, the baffles of the first baffle set and the baffles of the second baffle set may have different arrangement intervals.

According to an aspect, the baffles of the first baffle set and the baffles of the second baffle set may have different lengths.

According to an aspect, the baffle may include a first baffle member having one end portion fixed to the inner wall of the first channel or the second channel and another end portion extending toward a center line in the longitudinal direction of the first channel or the second channel and a second baffle member forming an angle with the other end portion of the first baffle member and further extending toward the center line, wherein the second baffle member prevents particles that exceed a predetermined size among the inhalable compositions from passing through the first channel or the second channel.

According to an aspect, the second baffle member may further extend beyond the center line of the first channel or the second channel.

According to an aspect, the cartridge may include a first receiving member communicating with the first channel and a second receiving member separated from the first receiving member by a partition wall and communicating with the second channel.

According to an aspect, the first receiving member may receive a first inhalable composition, and the second receiving member may receive a second inhalable composition having a different particle size or a different material from the first inhalable composition.

According to an aspect, the cartridge may be detachably fastened to the first surface by magnets or in a fitting manner.

According to an aspect, the first channel or the second channel may have a portion adjacent to the second surface and formed in a spiral shape.

### EFFECTS OF THE INVENTION

An inhaler according to an embodiment has an effect of transferring dry powder by utilizing an inertial force of particles and preventing inflow of particles of an unspecified size that may cause coughing.

An inhaler according to an embodiment has an effect of transferring a plurality of dry powders by arranging, on both sides, baffle structures with different performances of removing particles.

The effects of the inhaler according to an embodiment are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 schematically illustrates an inhaler according to an embodiment.
FIG. 2 is a plan view of an inhaler according to an embodiment.
FIG. 3 is a front view of an inhaler according to an embodiment.
FIG. 4 is a bottom view of an inhaler according to an embodiment.

The accompanying drawings illustrate preferred embodiments of the present invention and are provided together with the detailed description for better understanding of the technical idea of the present invention. Therefore, the present invention should not be construed as being limited to the embodiments set forth in the drawings.

### BEST MODE FOR CARRYING OUT THE INVENTION

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the following description of the present embodiments, a detailed description of publicly known configurations or functions incorporated herein will be omitted when it is determined that the detailed description obscures the subject matters of the present embodiments.

In addition, the terms first, second, A, B, A, and B may be used to describe components of the embodiments. These terms are used only for the purpose of discriminating one component from another component, and the nature, the sequences, or the orders of the components are not limited by the terms. When one component is described as being "connected", "coupled", or "attached" to another component, it should be understood that one component may be connected or attached directly to the other component, and an intervening component may also be "connected", "coupled", or "attached" to the components.

The same name may be used to describe an element included in the embodiments described above and an element having a common function. Unless otherwise mentioned, the descriptions of the embodiments may be applicable to the following embodiments and thus, duplicated descriptions will be omitted for conciseness.

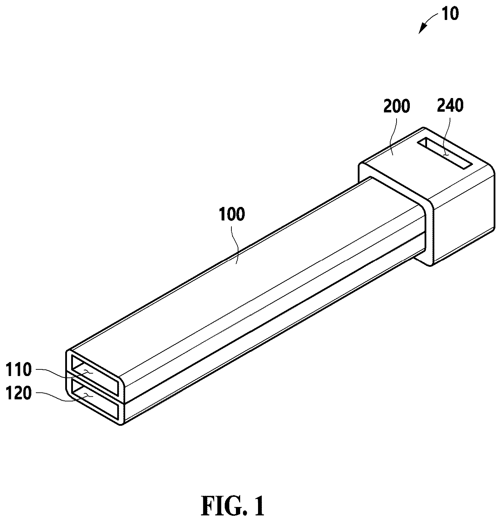

FIG. 1 schematically illustrates an inhaler 10 according to an embodiment.

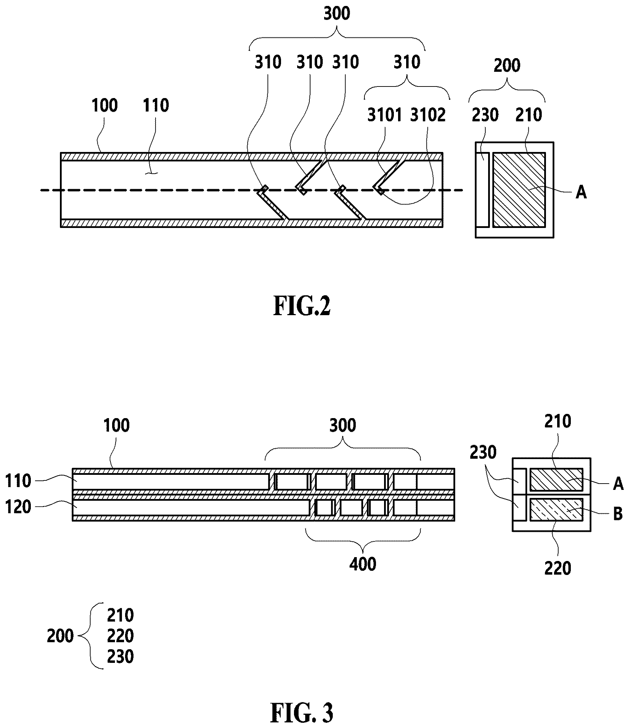

FIG. 2 is a plan view of the inhaler 10 according to an embodiment.

FIG. 3 is a front view of the inhaler 10 according to an embodiment.

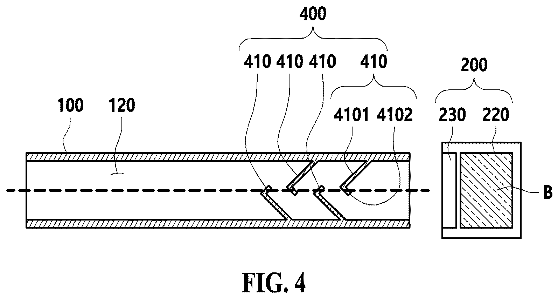

FIG. 4 is a bottom view of the inhaler 10 according to an embodiment.

Referring to FIG. 1, the inhaler 10 according to an embodiment may include a main body 100, a cartridge 200, a first baffle set 300, and a second baffle set.

The main body 100 may include a first surface, a second surface opposite to the first surface, and a plurality of side surfaces surrounding the first surface and the second surface. The cartridge 200 may be coupled to the first surface. A mouthpiece may be provided on the second surface. Channels 110 and 120 penetrating the first surface and the second surface may be formed inside the main body 100. The channels 110 and 120 may be passages through which an inhalable composition for transfer to a user is transferred to the mouthpiece. Here, the channels 110 and 120 may be partitioned into the first channel 110 and the second channel 120 along a longitudinal direction from the first surface to the second surface.

The first channel 110 or the second channel 120 may be formed in a spiral shape with an end portion that is adjacent to the second surface and has a spiral shape. Accordingly, a vortex may be generated in the flow of the inhalable composition.

In addition, the main body 100 may be provided with a flow distribution structure for controlling the degree of inhalation of the inhalable composition at an end portion adj acent to the second surface.

Furthermore, the main body 100 may include materials such as polylactic acid (PLA), acrylonitrile butadiene styrene copolymer (ABS), polycarbonate (PC), polypropylene (PP), etc. and may also include other biodegradable (eco-friendly) materials or metal.

The cartridge 200 may be coupled to the first surface of the main body 100. The cartridge 200 may contain the inhalable composition inside and may be connected internally with the channels 110 and 120 of the main body 100. The cartridge 200 may contain one or multiple doses of the inhalable composition. The inhalable composition may include substances such as nicotine, nicotine salts, caffeine, taurine, vitamins, etc. In addition, the inhalable composition may include flavored particles such as natural flavor particles, menthol, etc. The inhalable composition may be provided, for example, in dry powder form. Here, the dry powder used in the inhaler 10 may or may not have a carrier.

The cartridge 200 may contain a plurality of inhalable compositions.

Specifically, the cartridge 200 may include a first receiving member 210 and a second receiving member 220.

The first receiving member 210 and the second receiving member may each form a space inside the cartridge 200 and may be separated from each other by a partition wall.

The first receiving member 210 may communicate with the first channel 110 and may contain a first inhalable composition A.

The second receiving member 220 may communicate with the second channel 120 and may contain a second inhalable composition B. Here, the second inhalable composition B may have a different particle size from the first inhalable composition A. In addition, the second inhalable composition B may have a different composition material from the first inhalable composition A.

An air inlet 240 may be formed on one side of the cartridge 200. When a suction force is applied to the mouthpiece, air may be introduced into the first receiving member 210 or the second receiving member 220 through the air inlet 240, and the received inhalable composition may be transferred toward the mouthpiece along the channel with the flow of air.

As described above, the first inhalable composition A and the second inhalable composition B may each be transferred through different channels, and thus, the user may inhale a plurality of inhalable compositions A and B into the body with one inhaler 10.

In addition, the cartridge 200 may be detachably coupled to the first surface. Therefore, when the inhalable composition contained in the cartridge 200 is exhausted, a new cartridge 200 may be used as a replacement and mounted on the main body 100. Here, the cartridge 200 may be fastened to the main body 100 using magnets or in a fitting manner by having a groove 230 formed in a shape that matches the end portion of the main body 100.

A mesh net may be provided between the main body 100 and the cartridge 200. The mesh net may include the same material as a material of the main body 100, such as PLA, for example. In addition, the mesh net may include a chemically stable metal with no inhalation toxicity, such as stainless steel, for example.

Furthermore, the inhaler 10 according to an embodiment may be a device that transfers the inhalable compositions A and B including dry powder and the like by utilizing an inertial force of particles of the inhalable compositions A and B, and when the particles of the transferred inhalable compositions are larger than a certain size, the inhaler 10 may cause the user to cough. Accordingly, a plurality of baffles 310 and 410 may be provided in a zigzag structure as a filter structure to prevent inflow of particles of an unspecified size.

In addition, the inhaler 10 according to an embodiment may be a device that may transfer the plurality of inhalable compositions A and B and may transfer a plurality of dry powders by arranging, on both sides of the main body 100, a plurality of filter structures with different performances of removing particles.

The first baffle set 300 may be arranged in the first channel 110.

The second baffle set 400 may be arranged in the second channel 120.

When a suction force is applied to the mouthpiece, the inhalable compositions A and B contained in the cartridge 200 may pass through the channels and may be transferred toward the mouthpiece. Here, the first baffle set 300 and the second baffle set 400 may each control the particle size of the inhalable compositions transferred to the mouthpiece.

For example, the first baffle set 300 may filter particles larger than or equal to a predetermined size from the first inhalable composition A passing through the first channel 110. The second baffle set 400 may filter particles larger than or equal to a predetermined size from the second inhalable composition B passing through the second channel. Here, the predetermined particle sizes filtered by the first baffle set 300 and the second baffle set 400 may be different.

The first baffle set 300 or the second baffle set 400 may each include a plurality of baffles.

The plurality of baffles 310 of the first baffle set 300 may be arranged to be spaced apart alternately on an inner wall of the first channel 110 in the longitudinal direction, as shown in FIG. 2.

The plurality of baffles 410 of the second baffle set 400 may be arranged to be spaced apart alternately on an inner wall of the second channel 120 in the longitudinal direction, as shown in FIG. 4.

Here, as may be identified by comparing FIG. 2 to FIG. 4, the baffles 310 of the first baffle set 300 and the baffles 410 of the second baffle set 400 may have different arrangement intervals.

In addition, by comparing FIG. 2 to FIG. 4 again, the baffles 310 of the first baffle set 300 and the baffles 410 of the second baffle set 400 may have different lengths.

Hereinafter, the structure of the baffles 310 or the baffles 410 is described in detail using the baffles 310 of the first baffle set 300 as an example.

The baffles 310 may include a first baffle member 3101 and a second baffle member 3102.

The first baffle member 3101 may have one end portion fixed to the inner wall of the first channel 110 or the second channel 120, and the other end portion may extend toward a center line in the longitudinal direction of the first channel 110 or the second channel 120.

The second baffle member 3102 may further extend toward the center line while forming an angle with the other end portion of the first baffle member 3101. The second baffle member 3102 may further extend beyond the center line of the first channel 110 or the second channel 120. This second baffle member 3102 may not allow particles exceeding a predetermined size among the inhalable compositions to pass through the first channel 110 or the second channel 120.

As described above, by arranging, in one main body 100, the plurality of baffle sets 300 and 400 having different performances of removing particles, the inhaler 10 according to an embodiment may easily transfer the inhalable compositions A and B of different sizes.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a main body that comprises a first surface and a second surface facing the first surface and having a mouthpiece and has a first channel and a second channel formed inside and partitioned in a longitudinal direction from the first surface toward the second surface;
a cartridge coupled to the first surface of the main body, communicating with the first channel and the second channel, and containing a plurality of inhalable compositions; and
a first baffle set arranged in the first channel and a second baffle set arranged in the second channel,
wherein, when a suction force is applied to the mouthpiece, the first baffle set and the second baffle set control a particle size of the plurality of inhalable compositions transferred to the mouthpiece.

2. The inhaler of claim 1, wherein
the first baffle set or the second baffle set each includes a plurality of baffles,
wherein the plurality of baffles of the first baffle set is arranged to be spaced apart alternately on an inner wall of the first channel in the longitudinal direction, and
the plurality of baffles of the second baffle set is arranged to be spaced apart alternately on an inner wall of the second channel in the longitudinal direction.

3. The inhaler of claim 2, wherein the baffles of the first baffle set and the baffles of the second baffle set have different arrangement intervals.

4. The inhaler of claim 2, wherein the baffles of the first baffle set and the baffles of the second baffle set have different lengths.

5. The inhaler of claim 2, wherein the baffle comprises:
a first baffle member having one end portion fixed to the inner wall of the first channel or the second channel and another end portion extending toward a center line in the longitudinal direction of the first channel or the second channel; and
a second baffle member forming an angle with the other end portion of the first baffle member and further extending toward the center line,
wherein the second baffle member prevents particles that exceed a predetermined size among the inhalable compositions from passing through the first channel or the second channel.

6. The inhaler of claim 5, wherein the second baffle member further extends beyond the center line of the first channel or the second channel.

7. The inhaler of claim 1, wherein the cartridge comprises:
a first receiving member communicating with the first channel; and
a second receiving member separated from the first receiving member by a partition wall and communicating with the second channel.

8. The inhaler of claim 7, wherein the first receiving member receives a first inhalable composition, and
the second receiving member receives a second inhalable composition having a different particle size or a different material from the first inhalable composition.

9. The inhaler of claim 1, wherein the cartridge is detachably fastened to the first surface by magnets or in a fitting manner.

10. The inhaler of claim 1, wherein the first channel or the second channel has a portion adjacent to the second surface and formed in a spiral shape.
